**Europäisches Patentamt**

**European Patent Office**

**Office européen des brevets**

(19)

(11) Veröffentlichungsnummer: **0 034 795**
**B1**

(12) # EUROPÄISCHE PATENTSCHRIFT

(45) Veröffentlichungstag der Patentschrift:
03.07.85

(21) Anmeldenummer: 81101126.1

(22) Anmeldetag: 17.02.81

(51) Int. Cl.⁴: **G 03 F 7/26**, G 03 F 7/02,
C 25 D 1/10, B 01 D 59/18

(54) **Verfahren zum Herstellen von Lackschichten für die Mikrogalvanoplastik mittels Röntgenstrahlen.**

(30) Priorität: 21.02.80 DE 3006543

(43) Veröffentlichungstag der Anmeldung:
02.09.81 Patentblatt 81/35

(45) Bekanntmachung des Hinweises auf die Patenterteilung:
03.07.85 Patentblatt 85/27

(84) Benannte Vertragsstaaten:
**DE NL**

(56) Entgegenhaltungen:
**AT - B - 245 902**

(73) Patentinhaber: **Siemens Aktiengesellschaft, Berlin und München Wittelsbacherplatz 2, D-8000 München 2 (DE)**

(72) Erfinder: **Tischer, Peter, Dr.rer.nat.Phys.,
Roemerstrasse 16, D-8021 Strasslach (DE)**
Erfinder: **Glashauser, Walter, Paul-Klee-Strasse 4,
D-8000 München 71 (DE)**

## Beschreibung

Die vorliegende Patentanmeldung betrifft ein Verfahren zum Herstellen von Lackschichten für die Mikrogalvanoplastik mittels Röntgenstrahlen.

Für die Mikrogalvanoplastik mittels Röntgenstrahlen wird heute der Vorteil der Röntgenlithografie zur Herstellung von Mustern in Polymethylmetacrylaten (= PMMA) ausgenutzt. Dabei werden die entsprechenden Strukturen in einer etwa 10 µm dicken, auf einer Unterlage aus Metall, Silizium oder Glas befindlichen Lackschicht erzeugt, in denen dann galvanisch ein Metallmuster aufgebaut wird.

Will man beispielsweise für die Herstellung des Musters für die Trenndüsen einer Isotopen-Anreicherungsanlage die Strukturbreite auf 10 µm verkleinern und dabei die Lackschichtdicke auf 100 µm erhöhen, um in diesen Mustern dann galvanisch ein Metall aufzubauen, so treten bei der Entwicklung des Musters in der dickeren Lackschicht in den Bereichen, die nach der Entwicklung stehenbleiben, Sprünge und Risse auf, welche auf mechanische Spannungen beim Abkühlen der auf die Unterlage auflaminierten Lackschicht zurückzuführen sind. Diese Sprünge und Risse verhindern, dass ein einwandfreies Metallmuster durch galvanischen Aufbau erzeugt werden kann.

Aufgabe der Erfindung ist es daher, ein Verfahren anzugeben, bei dem beim Erzeugen einer mit 10 µm breiten Strukturen versehenen Lackschicht von ca. 100 µm Dicke bei der Durchführung der Röntgenlithografie keine Rissbildung in den nach der Entwicklung stehengebliebenen Bereichen auftritt.

Die erfindungsgemässe Aufgabe wird bei einem Verfahren der eingangs genannten Art dadurch gelöst, dass die Lackschicht auf einer mit einer Metallschicht versehenen Unterlage aus einem Material mit einem zumindest angenähert gleichen Ausdehnungskoeffizienten wie die Lackschicht erzeugt wird. Dabei liegt es im Rahmen des Erfindungsgedankens, dass bei der Herstellung einer aus Polymethylmetacrylat (PMMA) bestehenden Lackschicht eine Unterlage aus Polymethylmetacrylat verwendet wird, welche mit einer dünnen (< 1 µm, vorzugsweise 0,1 µm) Metallschicht versehen ist. Dabei ist es besonders vorteilhaft für die Metallschicht gut leitende und leicht galvanisierbare Metalle wie Kupfer oder Nickel zu verwenden.

Die Lackschicht kann sowohl als fertige Folie bei Temperaturen von 100 bis 150 °C auf die metallisierte Unterlage auflaminiert werden als auch aus einer Lösung durch Aufgiessen und Verdampfen des Lösungsmittels hergestellt werden.

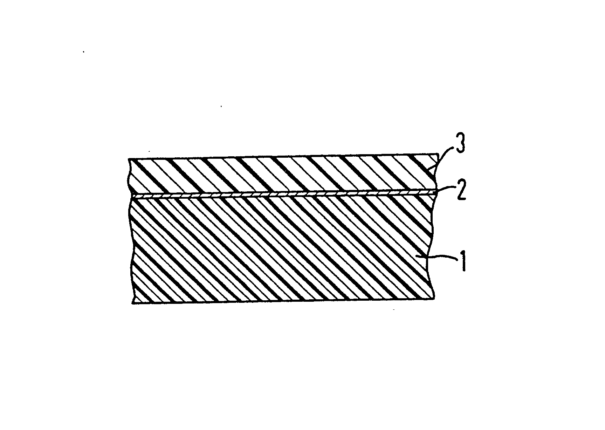

Weitere Einzelheiten sind der aus der Zeichnung befindlichen Figur sowie deren Beschreibung zu entnehmen.

Auf eine aus Plexiglas (= Polymethylmetacrylat = PMMA) bestehende Unterlage 1 von 2 mm Dicke wird eine 0,1 µm dicke Nickelschicht 2 aufgebracht (Dampfen, Sputtern, Sprühen). Dann wird bei 150 °C eine aus PMMA bestehende, 100 µm dicke Folie 3 auf die mit der Nickelschicht 2 versehene Plexiglasunterlage 2 auflaminiert und eine feste Haftung zwischen der Unterlage 1 und der Folie 3 unter Zwischenschaltung der Metallschicht 2 hergestellt. Um die PMMA-Schicht 3 für Röntgenstrahlen empfindlich zu machen, wird die Anordnung (1, 2, 3) auf 170 °C aufgeheizt und nach dem Abkühlen entsprechend der gewünschten Struktur belichtet und entwickelt. Dabei zeigt sich, dass infolge des gleichen Ausdehnungskoeffizienten von Unterlage und Lackschicht keine mechanischen Spannungen in der Lackschicht entstehen. Die dünne Metallschicht stört dabei nicht.

Nach dem Entfernen der Unterlage erfolgt der Vorgang der Galvanoplastik in bekannter Weise, wobei die Anordnung (2, 3) in ein galvanisches Bad aus einer Nickelsulfatlösung gebracht und als Kathode geschaltet wird. Hat der Nickelniederschlag die gewünschte Dicke (für die Fertigung von Trenndüsen etwa 100 µm) erreicht, dann wird die PMMA-Struktur weggelöst, so dass eine aus Metall bestehende Matrize erhalten wird, welche dann gestapelt und im Falle der Herstellung von Trenndüsen für Isotopenanreicherungsanlagen in Pakete bis 3 mm Dicke gepresst wird.

## Patentansprüche

1. Verfahren zum Herstellen von Lackschichten für die Mikrogalvanoplastik mittels Röntgenstrahlen, dadurch gekennzeichnet, dass die Lackschicht auf einer mit einer Metallschicht versehenen Unterlage aus einem Material mit einem zumindest angenähert gleichen Ausdehnungskoeffizienten wie die Lackschicht erzeugt wird.

2. Verfahren nach Anspruch 1, dadurch gekennzeichnet, dass bei der Herstellung einer aus Polymethylmetacrylat bestehenden Lackschicht eine Unterlage aus Polymethylmetacrylat verwendet wird.

3. Verfahren nach Anspruch 1 und/oder 2, dadurch gekennzeichnet, dass die aus einem gut galvanisierbaren Metall wie Kupfer oder Nickel bestehende Metallschicht in einer Dicke von < 1 µm, vorzugsweise 0,1 µm, aufgebracht wird.

4. Verfahren nach einem der Ansprüche 1 bis 3, dadurch gekennzeichnet, dass die Lackschicht als Folie bei Temperaturen von 100 bis 150 °C auf die Unterlage auflaminiert wird.

5. Verfahren nach einem der Ansprüche 1 bis 3, dadurch gekennzeichnet, dass die Lackschicht aus einer Lösung durch Aufgiessen auf die Unterlage und Verdampfen des Lösungsmittels hergestellt wird.

6. Verfahren nach Anspruch 5, dadurch gekennzeichnet, dass bei Verwendung von Polymethylmetacrylat als Lösungsmittel Chlorbenzol verwendet wird.

7. Verfahren nach einem der Ansprüche 1 bis 6, dadurch gekennzeichnet, dass die auf der Unterlage befindliche Lackschicht vor der Belichtung mit Röntgenstrahlen auf 170 °C aufgeheizt wird.

## Claims

1. A process for the production of varnish layers for microgalvanoplastics by means of X-rays, characterised in that the varnish layer is produced on a base which is provided with a metal layer and consists of a material having a coefficient of expansion which is at least approximately equal to that of the varnish layer.

2. A process as claimed in Claim 1, characterised in that, for the production of a varnish layer consisting of polymethyl methacrylate, a base made of polymethyl methacrylate is used.

3. A process as claimed in Claim 1 and/or Claim 2, characterised in that the metal layer, which consists of a highly electrodepositable metal such as copper or nickel, is applied in a thickness of < 1 μm, preferably 0.1 μm.

4. A process as claimed in one of Claims 1 to 3, characterised in that the varnish layer is laminated onto the base as a film at temperatures of 100 to 150°C.

5. A process as claimed in one of Claims 1 to 3, characterised in that the varnish layer is produced from a solution by pouring onto the base and evaporating the solvent.

6. A process as claimed in Claim 5, characterised in that when using polymethyl methacrylate, chlorobenzene is used as solvent.

7. A process as claimed in one of Claims 1 to 6, characterised in that, prior to exposure by means of the X-rays, the varnish layer which is located on the base is heated to 170°C.

**Revendications**

1. Procédé pour fabriquer des couches de vernis pour la microgalvanoplastie à l'aide de rayons X, caractérisé par le fait qu'on forme la couche de vernis sur un support muni d'une couche métallique et constituée en un matériau possédant un coefficient de dilatation au moins approximativement égal à celui de la couche de vernis.

2. Procédé suivant la revendication 1, caractérisé par le fait que, lors de la fabrication d'une couche de vernis constituée par du poly (méthacrylate de méthyle), on utilise un support en poly (méthacrylate de méthyle).

3. Procédé selon la revendication 1 et/ou 2, caractérisé par le fait que la couche métallique, qui est constituée en un métal pouvant être bien galvanisé, tel que du cuivre ou du nickel, est déposée sur une épaisseur inférieure à 1 μm, de préférence égale à 0,1 μm.

4. Procédé suivant l'une des revendications 1 à 3 caractérisé par le fait qu'on dépose sur le support la couche de vernis sous la forme d'une feuille plaquée, à des températures allant de 100 à 150°C.

5. Procédé suivant l'une des revendications 1 à 3, caractérisé par le fait qu'on réalise la couche de vernis à partir d'une solution par coulée sur le support et évaporation du solvant.

6. Procédé suivant la revendication 5, caractérisé par le fait que dans le cas d'utilisation de poly(méthacrylate de méthyle) on utilise comme solvant un chlorobenzène.

7. Procédé suivant l'une des revendications 1 à 6, caractérisé par le fait qu'on chauffe la couche de vernis située sur le support, à 170°C avant d'en réaliser l'exposition aux rayons X.